# EUROPÄISCHE PATENTSCHRIFT

(11) **EP 4 110 208 B1**
(45) Veröffentlichungstag und Bekanntmachung des Hinweises auf die Patenterteilung: **23.07.2025**
(21) Anmeldenummer: 21706310.6
(22) Anmeldetag: 24.02.2021
(51) Int. Cl.: A61B 17/72, A61F 2/28

(54) **EXTRAKORPORAL LÄNGENVERSTELLBARES IMPLANTATSYSTEM SOWIE LÄNGENVERSTELLBARE IMPLANTATKOMPONENTE**
EXTRACORPOREALLY LENGTH-ADJUSTABLE IMPLANT SYSTEM AND LENGTH-ADJUSTABLE IMPLANT COMPONENT
SYSTÈME D'IMPLANT À RÉGLAGE EN LONGUEUR EXTRACORPOREL AINSI QUE COMPOSANT D'IMPLANT RÉGLABLE EN LONGUEUR

(30) Priorität: 25.02.2020 EP 20159426
(43) Veröffentlichungstag der Anmeldung: 04.01.2023
(60) Teilanmeldung: 23201401.9
(73) Patentinhaber: Waldemar Link GmbH & Co. KG, 22339 Hamburg (DE)
(72) Erfinder: FISCHER, Hans-Joachim, 22850 Norderstedt (DE); LINK, Helmut D., 22397 Hamburg (DE); DMUSCHEWSKY, Klaus, 22339 Hamburg (DE)
(74) Vertreter: Alatis
(86) Internationale Anmeldenummer: PCT/EP2021/054500
(87) Internationale Veröffentlichungsnummer: WO 2021/170612

(56) Entgegenhaltungen:
- EP-A2- 1 611 868
- CN-A- 102 895 048
- US-A1- 2004 030 395
- US-A1- 2011 230 883
- US-A1- 2017 049 489
- US-B1- 7 753 915

## Beschreibung

Die Erfindung betrifft ein von außen (extrakorporal) verstellbare Implantatkomponente als Teil einer Endoprothese sowie ein Implantatsystem umfassend eine solche Implantatkomponente und eine Verstelleinrichtung dafür.

Die Verstelleinrichtung ist vorgesehen für einen Prothesenschaft der Implantatkomponente, die zur Befestigung an einem zu verlängernden Knochen ausgebildet ist, und umfasst gegeneinander verschiebliche Befestigungsteile, von denen jedes an einem Teil des zu verlängernden Knochens anzuordnen ist, sowie ein von einem Antriebselement angetriebenes Verstellelement, das dazu ausgebildet ist die zwei gegeneinander verschieblichen Befestigungsteile zu distrahieren.

Intrakorporal verstellbare Prothesen haben Bedeutung für An wendungsfälle, bei denen Prothesen sozusagen mitwachsen sollen. Sie sind insbesondere in solchen Fällen indiziert, wo entweder aufgrund natürlicher Vorgänge, wie beispielsweise beim Wachstum von Kindern, eine entsprechend längere Prothese benötigt wird, aber auch für solche Anwendungsfälle, bei denen zur Knochenbildung (Osteogenese) der Knochen entlang einer Trenn- bzw. Bruchstelle zusehends weiter gestreckt wird, um so fortlaufend die Bildung neuer Zellen anzuregen. Beispielsweise werden bei letzterem Anwendungsfall die zwei durch die Trenn stelle entstanden Knochenteile sukzessive voneinander wegbewegt, beispielsweise um 1 mm pro Tag, um so ständig die Bildung von neuem Knochengewebe anzuregen. Auf diese Weise kann beispielsweise eine Extremität, wie ein Bein, verlängert werden. Das ist von Bedeutung, um einen Längenausgleich zwischen den beiden Beinen eines Menschen herzustellen. In ähnlicher, wenn auch in der Regel deutlich langsamerer Weise, kann auch durch beständiges Erhöhen der Länge einer Prothese erreicht werden, dass der mit der Prothese versorgte Knochen in einer ähnlichen Weise länger wird, wie es beim natürlichen Wachstumsprozess von Kindern oder Jugendlichen der Fall wäre.

Bereits seit langem ist bekannt, dass für solche Fälle externe Fixateure verwendet werden. Diese weisen jedoch gravierende Nachteile auf, beispielsweise sind sie unhandlich sowie unfallgefährlich, und ferner haben sie ein hohes Risiko für Infektionen.

Um diesen Nachteilen zu begegnen, sind implantierbare Prothesen entwickelt worden, die eine gewünschte Längenverstellung mittels einer intrakorporal implantierten Prothese ermöglichen. Die eigentliche Längenverstellung kann hierbei invasiv erfolgen, insbesondere bei einer rein mechanischen Längenverstellung, oder mittels einem an der Prothese implantierten An trieb, der von außen ferngesteuert wird (Mutars-System der deutschen Firma Implantcast GmbH, Synoste-System der finnischen Firma Synoste Oy).

Letztere bieten den Vorteil, dass die Verstellung non-invasiv von außendurchgeführt werden kann. Dazu enthält die Prothese einen Elektromotor bzw. bildet einen Teil eines Elektromotors, der von außen (extrakorporal) angesteuert werden kann. Ggf. kann er auch von außen mit Energie versorgt werden. So ist ein solcher Aufbau mit einem sozusagen verteilten Elektromotor bekannt, bei dem die Prothese und ihr Verstellelement ein Teil eines Elektromotors bildet (sozusagen den Rotor), und der andere Teil mit der Erregung (sozusagen der Stator) von einem extrakorporale Aufsatz gebildet ist (WO 01/786141). Der eigentliche Elektromotor ist also im Grunde teilweise intrakorporal (mit seinem Rotor) und teilweise extrakorporal (mit seinem Stator) angeordnet.

Solch ein elektromotorbasiertes System ist einfach zu betätigen, allerdings ist auch eine Fehlbedienung verhältnismäßig leicht möglich. Somit kann eine ausreichende extrakorporale Verstellbarkeit erreicht werden, jedoch ist wegen der Gefahr eines Bedienfehlers, wobei der Elektromotor mit großer Verstellkraft eine für den Patienten gefährliche Fehlverstellung bewirkt, sachkundiges Personal zur Bedienung erforderlich. Die Zulassung eines solchen Systems ist daher aufwendig und schwierig. Die Anwendung kann meist nur durch Fachpersonal er folgen, was zusätzlichen Aufwand verursacht. Dies führt zu Abstimmungsproblemen in Bezug auf die erforderlichen Termine, an denen das Fachpersonal Zeit hat für die Behandlung (ambulanter) Patienten, oder führt zu (über)großen Verstelleinheiten (in cm Verstellweg je Termin), um die Anzahl der Besuche des Patienten zu minimieren (wenn auch um den Preis von medizinisch eventuell fragwürdig großen Verstelleinheiten je Ter min).

Die US2011/230883 A1 offenbart ein längenverstellbares IM-Nagelsystem, dass einen teleskopierbaren IM-Nagel mit einem proximalen und einem distalen Körper umfasst. Ein Innenmagnet im proximalen Körper ist mit einer Gewindestange verbunden, die ihrerseits mit dem distalen Körper verbunden ist. Die Gewindestange führt durch einen Gewindeblock, der mit dem proximalen Körper verbunden ist. Die Position des distalen Endes der Gewindestange ist in Bezug auf den distalen Körper fixiert, kann sich aber innerhalb dieser fixierten Position frei drehen. Es wird auch ein Aktuator offenbart, der ein Paar rotierende Magnete umfasst, die in einem Winkelverhältnis zueinander und zur Achse des IM-Nagels und der Gliedmaße des Patienten angeordnet sind. Die Drehung dieser äußeren Magnete in dieselbe Richtung führt zu einer Drehung des inneren Magneten und der Gewindestange sowie zu einer axialen Teleskopbewegung des Gewindestücks und des proximalen Körpers in Bezug auf den distalen Körper.

Die CN 102 895 048 A bezieht sich auf einen künstlichen Knochen für einen menschlichen Körper, der in der Lage ist, die Länge durch ein nicht-chirurgisches Verfahren zu verlängern. Der künstliche Knochen ist dadurch gekennzeichnet, dass er einen oberen Knochen, einen unteren Knochen, einen Drehkörper, eine Schutzabdeckung und einen Steuerabschnitt umfasst, wobei das untere Ende des oberen Knochens einstückig einen ersten spiralförmigen Verbindungsabschnitt bildet, das obere Ende des unteren Knochens einstückig einen zweiten spiralförmigen Verbindungsabschnitt bildet und der gedrehte Körper mit dem ersten Spiralverbindungsabschnitt und dem zweiten Spiralverbindungsabschnitt verbunden ist und sich dreht, um die Trennung des oberen Knochens vom unteren Knochen zu ermöglichen. Magnetische Ringe sind regelmäßig am Umfang des gedrehten Körpers verteilt. Die Schutzabdeckung erstreckt sich, um den gedrehten Körper entlang der Längsrichtung vollständig zu umschließen, um das menschliche Körpergewebe davor zu bewahren, mit der Außenseite in Kontakt zu kommen. Der Steuerabschnitt ermöglicht eine Drehung des gedrehten Körpers durch magnetische Kraft.

Aus der US 7 753 915 B1 ist ein System zur Einstellung der Knochenlänge bekannt, das eine zweiteilige teleskopische Vorrichtung umfasst, die mit Knochenteilen verbunden ist, deren Länge zwischen den Verbindungspunkten eingestellt werden soll. Jedes Teil der teleskopischen Vorrichtung ist an einem Knochenteil befestigt. Eine Stange mit einem eingebetteten Permanentmagneten, steht an beiden Enden in Gewindeeingriff mit beiden Teilen der teleskopischen Vorrichtung. Der Permanentmagnet wird durch eine externe Magnetanordnung erregt, so dass sich an der Stange eine Radialkraft entwickelt, die sie in Drehung versetzt. Die Drehung der Stange wird über die Gewinde an beiden Enden in eine axiale Bewegung der Teleskopteile relativ zueinander umgewandelt und bewirkt dadurch eine Zunahme oder Abnahme der Knochenlänge in Abhängigkeit von der Richtung der axialen Bewegung zwischen den Teleskopteilen.

US 2004/030395 A1 offenbart eine chirurgische Distraktionsvorrichtung, um nicht-invasiv eine Streck- oder Spannkraft auf das Skelett eines Patienten oder auf ein Implantat auszuüben, die Verankerungsmittel zum Befestigen einer ersten und einer zweiten Komponente der Vorrichtung an einem Knochen oder an angrenzenden Knochen umfasst, wobei die Komponenten durch ein Gestänge mit einer streckbaren Länge verbunden sind. Die Distraktionsvorrichtung weist einen Magneten, der mit dem Gestänge über ein Untersetzungsgetriebe verbunden ist, und Betätigungsmittel auf, die außerhalb des Patienten angeordnet sind, um ein sich bewegendes oder variierendes elektromagnetisches Feld zu erzeugen, wodurch der Magnet zum Drehen und das Gestänge zum Strecken veranlasst wird.

Der Erfindung liegt die Aufgabe zugrunde, ein verbessertes System zu schaffen, das eine extrakorporale Betätigung erlaubt und einfacher in der Handhabung ist.

Die erfindungsgemäße Lösung liegt in den Merkmalen des unabhängigen Anspruchs. Vorteilhafte Weiterbildungen sind Gegen stand der abhängigen Ansprüche.

Die Erfindung bezieht sich auf eine Implantatkomponente, bei der die Verstelleinrichtung so beschaffen ist, dass ein Antriebselement mit einem drehbeweglich angeordneten Permanentmagnet über ein Getriebe auf einen Spindeltrieb wirkt zum Umwandeln einer Drehbewegung des Permanentmagnets in eine distrahierende Längsbewegung, wobei das Antriebselement zur Betätigung mittels eines Magnetfelds einer extrakorporalen Antriebseinheit ausgebildet ist, und mit besonderem Vorteil zur Leistungsübertragung von dem Antriebselement auf den Spindel trieb ein Biegestab vorgesehen ist.

Dank des Biegestabs, der langgestreckt und zur Aufnahme bzw. Übertragung von Scherkräften ausgebildet ist, kann eine unter Last entstehende Verbiegung der Implantatkomponente ausgeglichen werden. Der Biegestab ermöglicht es, dass ein Winkelfehler zwischen dem Antriebselement mit dem Getriebe (Reduktionsgetriebe) einerseits und dem eigentlichen Spindeltrieb andererseits ausgeglichen werden kann. Ein solcher Winkelfehler entsteht insbesondere dadurch, dass die Implantatkomponente sich unter hoher Lastbeanspruchung verbiegt. Die sich relativ zueinander bewegenden Befestigungsteile, typischerweise Innen- und Außenrohr eines teleskopartigen Verstellmechanismus, werden dabei unter Last quer zu ihrer Mittelachse verbogen. Es besteht dann die Gefahr, dass das eigentliche Antriebselement mit dem Getriebe bezüglich seiner Achse nicht mehr exakt fluchtet mit der Achse des Spindeltriebs. Solche Fluchtungsfehler, die meist nur auf geringen Winkeldifferenzen beruhen, können zu einem Blockieren des Mechanismus führen. Die Erfindung hat erkannt, dass dies mittels eines Biegestabs zwischen Antriebselement und Getriebe einerseits und Spindeltrieb andererseits auf verblüffend einfache Weise zuverlässig verhindert werden kann. So kommt es auch unter hoher Last nicht zu einer Gefahr des Blockierens des Verstellelements.

Zweckmäßigerweise weist dazu die Antriebsspindel eine einseitig offene zentrische Bohrung auf, in die der Biegestab eingesteckt ist. Damit ist der Biegestab zum einen gut geführt, zum anderen ergibt sich bei kompaktem Aufbau eine verhältnismäßig große Länge für den Biegestab. Dies ermöglicht es, auch einigermaßen große Winkelfehler von bis zu gut +/- 5 Grad mittels des Biegestabs aufzunehmen. Zur kraftschlüssigen Verbindung zwischen Biegestab einerseits und der Antriebspindel andererseits ist vorzugsweise am Grund der zentrischen Bohrung eine Kopplung zwischen dem freien Ende des Biegestabs und der Antriebspindel vorgesehen. Dazu ist weiter zweckmäßigerweise vorgesehen, dass der Biegestab eine Länge aufweist, die vor zugsweise mindestens einem Drittel, weiter vorzugsweise mindestens einer Hälfte, der Länge der Antriebsspindel entspricht, und insbesondere dazu ausgebildet ist, einen Winkelversatz von bis zu +/- 5 Grad aufzunehmen. Mit Vorteil ist der Biegestab entlang seines Hauptteils seitlich eingeschnürt ausgeführt. Dies erleichtert zum einen das gewünschte Verbiegen des Biegestabs und sichert zum anderen den erforderlichen Freigang des Biegestabs in der zentrischen Bohrung.

Bei einem von extrakorporal längenverstellbarem Implantatsystem umfassend
- eine Implantatkomponente wie vorher beschrieben; sowie
- eine extrakorporale Antriebseinheit für das Antriebselement der Implantatkomponente, ist ferner vorgesehen, dass die extrakorporale Antriebseinheit einen drehbar gelagerten extrakorporalen Magnetring mit einem Innenraum, der zur Auf- nähme des zu verlängernden Knochens mit der Verstelleinrichtung ausgebildet ist, und eine Betätigungseinrichtung auf weist, die dazu ausgebildet ist, den extrakorporalen Magnet ring in seiner Ringebene zu rotieren, wobei der extrakorporale Magnetring in Permanentmagnetbauweise ausgeführt ist und in seinem Innenraum ein gerichtetes statisches magnetisches Feld aufweist, das ringfest ist, und der extrakorporale Magnetring mechanisch von der Betätigungseinrichtung rotiert ist.

Hier seien einige verwendete Begriffe erläutert:
Unter "ringfest" wird eine Anordnung verstanden, die fix in Bezug auf den Ring ist und mit dem Ring mitrotiert.

Unter "gerichtet" wird eine typischerweise einheitliche Ausrichtung eines Magnetfelds verstanden. Insbesondere soll die Ausrichtung des Magnetfelds an jeder Stelle des Feldes die gleiche Richtung aufweisen. Als "homogen" wird ein im wesentlichen gleichförmiges Magnetfeld bezeichnet, bei dem lokale Abweichungen der Feldstärke in einem mindestens 80% des Gesamtgebiets umfassenden Zentralgebiet höchstens +/- 10% vom Mittelwert betragen.

Unter "statisch" wird ein Magnetfeld von gleichbleibender Stärke verstanden. Es bildet so insbesondere einen Gegensatz zu den sich typischerweise schnell ändernden elektromagnetischen Feldern.

Diese Ausführungsform der Erfindung fußt auf dem Gedanken, sich von dem elektromotorisch betätigten Konzept zu lösen und stattdessen eine mechanische Betätigung vorzusehen. Die Erfindung erreicht dies, indem sie mittels des Magnetrings ein statisches Magnetfeld bereitstellt, das mechanisch (manuell oder motorisch) gedreht werden kann. Vorzugsweise wird der Magnetring als Ganzes von der Betätigungseinrichtung rotiert. Der innerhalb der Implantatkomponente als Antriebselement fungierende Permanentmagnet richtet sich stets gemäß dem statischen Magnetfeld des Magnetrings aus. Die Stellung des als Antriebselement fungierenden Permanentmagneten innerhalb der Verstelleinrichtung ergibt sich also aus der Position bzw. der erfolgten Drehung des statischen Magnetfelds. Mit dem Übergang auf ein mechanisch gedrehtes statisches Magnetfeld erreicht die Erfindung eine bedeutende Vereinfachung und eine genaue Positionskontrolle über die Position des als Antriebselement fungierenden Permanentmagneten. So kann eine simple manuelle Betätigung genügen; Strom zur Betätigung wird nicht mehr benötigt.

Damit entfällt auch jede Gefahr eines elektrischen Schlages durch eine Fehlfunktion. Ferner entfällt auch jedes Risiko, dass es durch eine Fehlfunktion zu einem Durchgehen bzw. Weg laufen der Positionsverstellung kommen kann, was zu einer übergroßen Verstellung und damit zu einer Verletzung des Knochens selbst und insbesondere seiner umgebenden Weichteile, wie Muskeln oder Sehnen, führen kann. Außerdem wird dank des gleichbleibenden statischen Magnetfelds eine Belastung des Patienten durch Wirbelströme, wie sie erzeugt werden durch sich ändernde elektromagnetische Felder, vermieden.

Das System mit seiner auf statischem Magnetfeld beruhenden Antriebseinheit kann somit auch von Hilfskräften bzw. Laien bedient werden, im Grunde auch von dem Patienten selbst. Dies stellt in der medizinischen Praxis einen erheblichen Vorteil dar, da somit der Patient selbst zu Hause bei Bedarf die Verstelleinrichtung betätigen kann. So ist es ermöglicht, die Verstellung häufiger und dann in schonenderen kürzeren Abschnitten vorzunehmen, als es bisher im Stand der Technik möglich war, insbesondere wenn eine Bedienung nur durch eingewiesene Fachpersonal erfolgen durfte.

Ferner erlaubt diese Ausführungsform der Erfindung mit dem drehbaren Magnetring eine präzise Kontrolle über die Ausrichtung des statischen Magnetfelds. Es kann so eine Feineinstellung der Winkelposition des Antriebselements der Verstelleinrichtung erreicht werden, was eine sehr genaue Einstellung der erreichten Verstellung ermöglicht.

Die Erfindung erreicht so auf verblüffend einfache Weise eine Erhöhung der Verstellsicherheit bei gleichzeitiger Vereinfachung der Antriebseinheit, was sie somit laien- und patiententauglich macht.

Vorzugsweise ist der Magnetring teilbar in mindestens zwei Segmente, um so einen öffenbaren und verschließbaren Zugang für ein mit dem Prothesenschaft versehenes Körperteil, insbesondere Bein oder Arm, zum Innenraum des Magnetrings zu schaffen. Damit können der Magnetring und damit die Antriebseinheit als Ganzes auf einfache Weise an der Stelle angeordnet werden, an der die Implantatkomponente mit der Verstelleinrichtung im Körper angeordnet ist. Beispielsweise kann durch Aufklappen des Magnetrings die Antriebseinheit ohne weiteres im Bereich des Oberschenkels eines Patienten positioniert werden, um die in dem Schaft einer im Femur angeordneten Implantatkomponente zu verstellen. Ein Durchfädeln der zu behandelnden Extremität durch den Magnetring entfällt damit. Vorzugsweise ist der Rest der Antriebseinheit so gestaltet, dass mit dem Aufklappen des Magnetrings ein freier Zugang zu dessen Innenraum ermöglicht ist. Die Anwendung ist dank des aufklappbaren Magnetrings so mit beträchtlich erleichtert.

Zweckmäßigerweise ist ferner vorgesehen, dass das Aufklappen des Magnetrings durch die Magnetkraft der in dem Magnetring angeordneten Magnete weder verhindert noch gefördert wird. Dazu ist der Magnetring so gestaltet, dass er magnetkraftfrei aufklappbar ist. Unter "magnetkraftfrei" wird hierbei verstanden, dass die von der Magnetkraft gegen ein Aufklappen gerichtete Kraft entweder Null oder so gering ist, dass sie ein Bediener von Hand ohne weiteres, insbesondere ohne Hinzunahme von Werkzeug, überwinden kann. Mit Vorteil ist dazu eine Trennebene zwischen den Segmenten so gewählt, dass sie sich in Richtung des gerichteten statischen magnetischen Felds er streckt. Damit wird erreicht, dass quer zur Trennebene laufende Anteile der Magnetkraft minimiert sind.

Hierbei sind vorzugsweise die Segmente mit Verbindungsmitteln verbunden, von denen mindestens eines offen- und wiederverschließbar und mindestens eines scharnierartig klappbar ist. Dank dieser Verbindungsmittel können die Segmente leicht geöffnet werden und nach Platzieren der betroffenen Extremität (das ist derjenige Körperteil, in dem die Implantatkomponente mit der erfindungsgemäßen Verstelleinrichtung enthalten ist, insbesondere ein Arm oder ein Bein) wieder geschlossen werden. In dem eines der Verbindungsmittel eine scharnierartige Klappbarkeit ermöglicht, kann ein vollständiges Abnehmen des öffenbaren Segments vermieden werden (obwohl auch das grundsätzlich möglich wäre). Die Handhabung wird damit weiter erleichtert.

Im geöffneten, aufgeklappten Zustand ist der Magnetring vor zugsweise gegen (unerwünschte) Bewegung gesichert. Dazu ist zweckmäßigerweise eine Arretiervorrichtung vorgesehen, die den Magnetring mit seinen Segmenten in der Öffnungsposition verdrehsicher fixiert. Damit wird dem Risiko einer ungeplanten Bewegung des Magnetrings, die auch zu einem unerwünschten Zu klappen führen könnte, auf wirksame Weise begegnet. Weiter vorzugsweise ist in sozusagen umgekehrten Sinne eine Sicherungseinrichtung vorgesehen, die insbesondere mittels einer Zwangsführung dazu ausgebildet ist, eine Koppelung des Magnet rings in der geschlossen Position zu bewirken. Es wird so ein Aufklappen des Magnetrings außerhalb der Öffnungsposition blockiert. Damit wird ein unerwünschtes Öffnen des Magnetrings beispielsweise während der normalen Betätigung, sicher verhindert.

Der Magnetring ist mit Permanentmagneten ausgeführt. Zweckmäßigerweise ist er gebildet mittels einer Vielzahl von Sub-Magneten, die an dem Magnetring angeordnet sind. Diese ermöglicht die Nutzung handelsüblicher und damit kostengünstig verfügbarer typischer Permanentmagnete als Sub-Magnete. So können auch verhältnismäßig große Magnetringe effizient und mit verhältnismäßig wenig Gewicht hergestellt werden. Die einzelnen Sub- Magnete, Einzelmagnete, sind vorzugsweise gleichartig, und zwar insbesondere als magnetische Dipolkörper ausgeführt, vor zugsweise aus Seltene Erde-Metalle (sog. Seltenerdmagnete). Die Sub-Magnete sind hierbei vorzugsweise entlang des Magnetrings regelmäßig mit verschiedener Feldorientierung winkelfest eingesetzt. Durch die winkelfeste Anordnung behalten die Sub- Magnete im Magnetring ihrer Feldorientierung stets bei. Indem die einzelnen Sub-Magnete mit voneinander verschiedener Feldorientierung eingesetzt sind, ist es möglich, einen solchen Feldverlauf des statischen Magnetfelds zu schaffen, dass sich eine Feldkonzentration im Inneren des Magnetrings ergibt und außerhalb des Magnetrings eine Feldauslöschung. Ferner ist es möglich, auf diese Weise ein homogenes Magnetfeld innerhalb des Magnetrings zu erzeugen. Ideal wird dies erreicht durch möglichst viele und kleine Submagnete, jedoch kann bereits mit einer Anzahl von Submagneten von gut einem Dutzend (10 bis 20) bereits eine gute Approximation für ein nahezu homogenes Feld im Magnetring erreicht werden.

Weiter sind die Sub-Magnete zweckmäßigerweise so angeordnet, dass sich ihr Magnetfeld (genauer gesagt die Streufelder außerhalb der Sub-Magnete) entlang einer Trennebene zwischen Segmenten des Magnetrings jeweils maximal kompensiert. Somit kann das Magnetfeld quer zur Trennebene minimiert werden. Das ist ein besonders vorteilhafter Weg, um auf einfache und zu verlässige Weise zu erreichen, dass die Segmente sich magnetkraftfrei öffnen lassen, wie vorstehend bereits ausgeführt.

Mit Vorteil ist ein Gehäuse für die Antriebseinheit vorgesehen, das den Magnetring umgibt. Mit dem Gehäuse werden magnetische Felder außerhalb des Magnetrings, insbesondere Streufelder, kurzgeschlossen, so dass der das Gehäuse umgebende Außenraum weitgehend frei von magnetischen Feldern ist ("außenfeldfrei"). Dazu weist das Gehäuse eine oder mehrere Schirmungen auf und/oder ist vorzugsweise aus weichmagnetischem Material gebildet. Somit ergibt sich nach außen keine Beeinflussung durch das (im Innenraum recht starke) Magnetfeld des Magnetrings. Ein sicherer Transport wird damit deutlich erleichtert.

Zweckmäßigerweise ist die Antriebseinheit mit einer Positionierungseinrichtung versehen, die auf den Magnetring wirkt, insbesondere ein Umdrehungen des Magnetrings anzeigendes Zählwerk. Damit kann die Position des Magnetrings angezeigt werden, was dem Benutzer eine feine und zielgenaue Einstellung ermöglicht. Das gilt sowohl für die Winkelposition des Magnetrings wie auch für die Anzahl der Umdrehungen des Magnetrings. So kann dem Bediener beispielsweise mittels eines Zählwerks auf gut ablesbare und reproduzierbare Art eine Information über die Verstellstrecke der Verstelleinrichtung angezeigt werden, die mit der Antriebseinrichtung bewirkt worden ist.

Mit Vorteil kann ferner ein extrakorporaler Sperrschalter vorgesehen sein, der vorzugsweise an der Antriebseinheit angeordnet ist und auf ein an dem Prothesenschaft angeordnetes Verstellsperrorgan wirkt. Es kann so mittels des Verstellsperrorgans das Verstellelement der Implantatkomponente gegen unbeabsichtigte Verstellung gesperrt werden. Ist eine Verstellung mit der Antriebseinheit vorgesehen, wird das Verstellsperrorgan mittels des Sperrschalters gelöst, und damit das Verstellelement wieder freigegeben, um so die gewünschte Verstellung mittels der Antriebseinheit vornehmen zu können.

Die Antriebseinheit ist vorzugsweise mit einem Standfuß versehen. Er hält die Antriebseinheit in aufrechter Position und fungiert zugleich als Kippschutz, um so eine korrekte relative Positionierung von der Antriebseinheit zu der Implantatkomponente zu halten. Um eine initiale korrekte Positionierung zu erreichen, ist vorzugsweise der Standfuß ferner versehen mit einer richtungsorientierten Aufnahme für das Körperteil, insbesondere Arm oder Bein, in das die Implantatkomponente mit der Verstelleinrichtung implantiert ist.

Für weitere Angaben zum Zusammenwirken der Implantatkomponente mit der Antriebseinrichtung wird auf vorstehende Beschreibung verwiesen.

Die Erfindung wird nachfolgend unter Bezugnahme auf die beige fügte Zeichnung anhand vorteilhafter Ausführungsformen näher erläutert. Es zeigen:
- Fig. 1: eine schematische Ansicht eines Knochens mit ein- gesetzter Implantatkomponente sowie einer daneben angeordneten Antriebseinheit;
- Fig. 2: eine schematische Darstellung eines statischen Magnetfelds innerhalb eines Magnetrings;
- Fig. 3a, b: schematische Darstellung eines Sub-Magneten sowie physische Darstellung zweier benachbart angeordneter Sub-Magnete;
- Fig. 4: eine Frontalansicht einer ersten Ausführungsform einer Antriebseinheit mit einem Magnetring ;
- Fig. 5: eine Frontalansicht der ersten Ausführungsform in einem teilweise aufgeklappten Zustand;
- Fig. 6: eine perspektivische Ansicht einer zweiten Ausführungsform einer Antriebseinheit ;
- Fig. 7: eine Schnittansicht der zweiten Ausführungsform mit einem Schnitt durch das Gehäuse und Frontalsicht auf den Magnetring; Fig. 8 eine perspektivische Ansicht der zweiten Ausführungsform in einem teilweise aufgeklappten Zustand;
- Fig. 9: eine perspektivische Ansicht der zweiten Ausführungsform in einem voll aufgeklappten Zustand;
- Fig. 10: eine perspektivische Ansicht der zweiten Ausführungsform mit teilweise entferntem Gehäuseoberteil;
- Fig. 11: eine Frontalansicht als Schnitt durch das Gehäuse der in Figur 10 dargestellten Ausführungsform;
- Fig. 12a, b: weitere perspektivische Ansichten der zweiten Aus führungsform mit aufgesetztem Gehäuseoberteil und mit teilgeschnittener Verriegelung;
- Fig. 13a, b: eine Frontal- sowie eine Seitenansicht einer dritten Ausführungsform mit schwenkbarem Schutzdeckel;
- Fig. 14: eine perspektivische Ansicht der dritten Ausführungsform;
- Fig. 15: Darstellungen zur Abschirmung am Beispiel der ersten Ausführungsform; Fig. 16 eine perspektivische Darstellung einer Implantatkomponente mit Verstelleinrichtung ;
- Fig. 17: eine Aufsicht auf die Implantatkomponente mit freigelegtem Innenraum;
- Fig. 18: eine teilgeschnittene Darstellung der Implantatkomponente gemäß Figur 17; und
- Fig. 19: eine Schnittansicht einer Spindel für eine Ver Stelleinrichtung der Implantatkomponente.

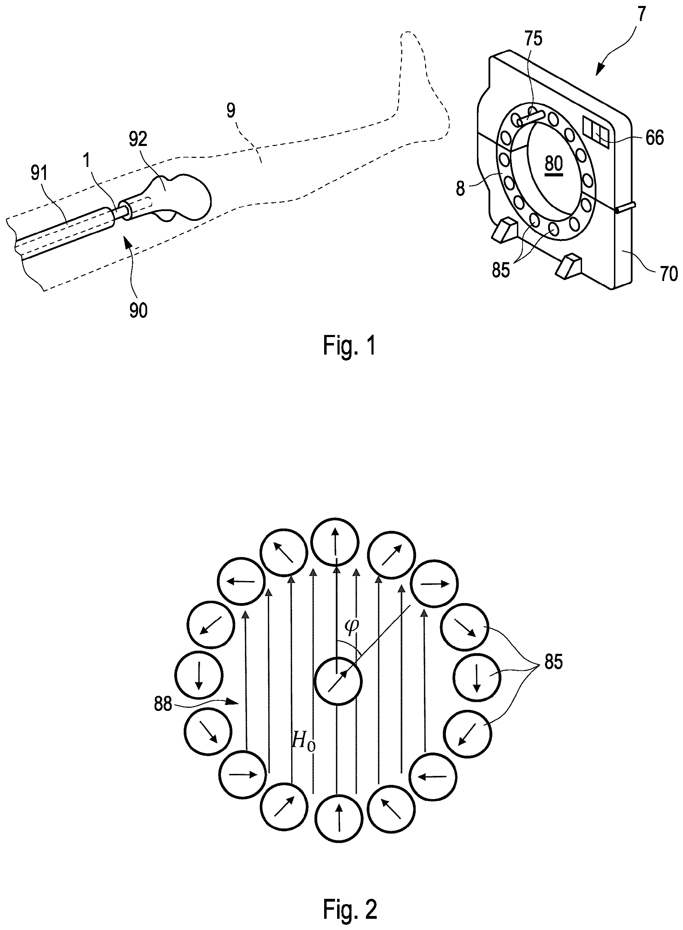

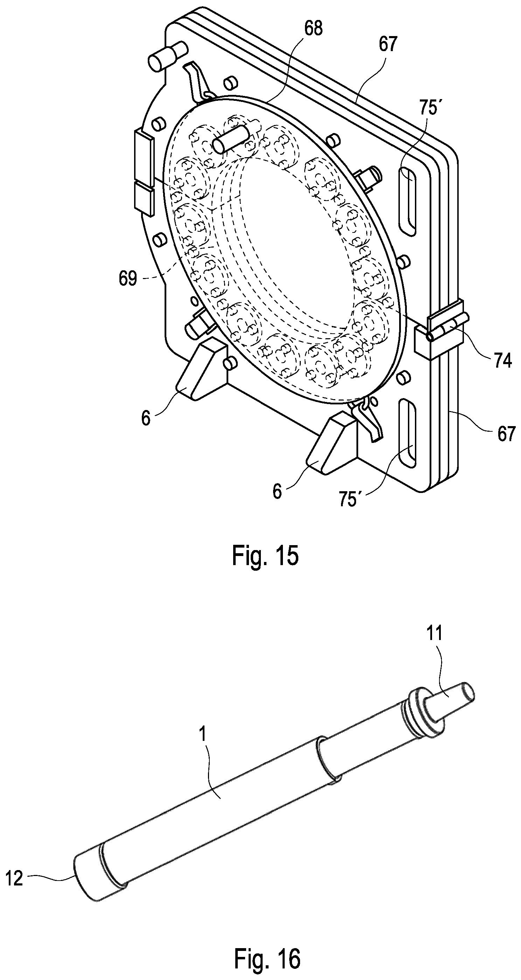

Ein System zur Längenverstellung einer implantierten Prothese sowie Verstelleinrichtung hierzu ist in Gestalt eines ersten Ausführungsbeispiels in Figur 1 schematisch dargestellt. Man erkennt eine untere Extremität, ein Bein 9, welches einen zu verlängernden Knochen 90 aufweist, in Gestalt eines Femurknochens. Er ist in zwei Teile 91, 92 geteilt, wobei in beiden Teilen die Implantatkomponente 1 verankert ist und somit die beiden Teile 91, 92 des Femurknochens 90 miteinander verbindet. Insbesondere kann es sich bei der Implantatkomponente 1 um einen längenverstellbaren Schaft eines Prothesensystems handeln, wie er beispielsweise in Figur 16 abgebildet ist.

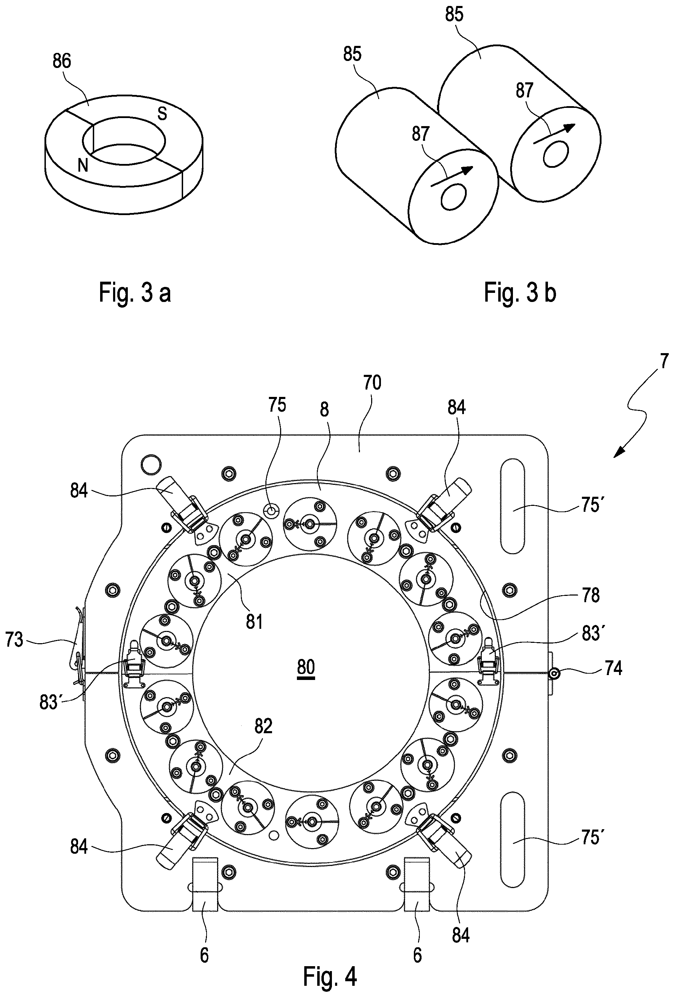

Zur Verbindung mit anderen Implantatkomponenten weist die erfindungsgemäße Implantatkomponente 1 an ihren beiden Enden jeweils einen Konusverbinder auf, vorzugsweise an einem Ende einen männlichen Konusverbinder 11 und an dem anderen Ende einen weiblichen Konusverbinder 12 (es versteht sich, dass auch andere Verbindungsformen möglich sind, bspw. mittels Gewinde etc.). Bei der Implantatkomponente kann es sich insbesondere um einen Teil einer größeren Gelenkendoprothese handeln, insbesondere für einen Totalersatz des Femurknochens 90. Mittels der Verstelleinrichtung 2 (in Figur 1 nicht dargestellt) können die Enden der Implantatkomponente 1 mit den Konusverbindern 11, 12 voneinander wegbewegt werden, d. h. die Länge der Implantatkomponente 1 kann verändert, insbesondere vergrößert werden. Zur Betätigung der Verstelleinrichtung 2 ist eine extrakorporale Antriebseinheit 7 vorgesehen. Sie umfasst einen Magnet ring 8, der drehbar innerhalb eines Trägers 70 gehaltert und geführt ist. Der Magnetring 8 ist so ausgeführt, dass in seinem Innenraum 80 ein gerichtetes statisches Magnetfeld entsteht. Ein solches gerichtetes statisches Magnetfeld 88 ist in Figur 2 dargestellt. Der Magnetring 8 ist hierbei nicht als ein unitärer Massivkörper ausgeführt, sondern ist vielmehr diskret aufgebaut, nämlich aus einer Vielzahl von Sub-Magneten 85. Die Sub-Magnete 85 sind jeweils als magnetischer Dipol ausgeführt, das bedeutet der eigentliche Magnetkörper weist auf der einen Seite einen Nordpol "N" auf der anderen Seite einen Südpol "S" auf (dies ist in Figur 3a durch unterschiedliche Grauschattierung kenntlich gemacht). Bildet man den dort dargestellten Magnetkörper weiter zu einer zylindrischen Form, so bleibt die Anordnung von Nordpol und Südpol im Grunde unverändert (dies ist in Figur 3b dargestellt, wobei die Richtung der remanenten Magnetisierung 87 durch einen schwarzen Pfeil hervorgehoben ist). Daraus ergibt sich weiterhin eine bestimmte magnetische Orientierung. Bei den Sub-Magneten 85 handelt es sich also technisch gesehen um Dipol-Magnete 86, die im Inneren ein gerichtetes magnetisches Feld aufweisen, wie in Figur 3b deutlich dargestellt ist.

Die Erfindung macht sich diese Charakteristik der Sub-Magnete 85 zu Nutze und schafft so mit einer überschaubaren Anzahl (nämlich 16) von Sub-Magneten 85 ein gerichtetes statisches Magnetfeld, das im Übrigen auch weitgehend homogen ist (siehe Figur 2). Hierbei wird unter "homogen" verstanden, dass über die Fläche des Innenraums 80 gesehen sich eine ortsabhängige Abweichung der magnetischen Feldstärke ergibt, welche nicht größer ist als +/- 15 % der tatsächlichen mittleren magnetischen Feldstärke. Erreicht wird dies durch eine bestimmte winkelmäßige Orientierung der Sub-Magnete 85 im Magnetring 8. Hierbei sind zum einen die Sub-Magnet 85 äquidistant zum Mittelpunkt angeordnet, d. h. es ergibt sich idealerweise ein gleichmäßiger Kreis. Zum anderen sind die Sub-Magnete 85 mit unterschiedlicher Orientierung in den Magnetring 8 eingesetzt.

Um dies zu visualisieren, ist in Figur 2 (in entsprechender Weise wie bei Figur 3b) die Richtung 87 der remanenten Magnetisierung eingezeichnet. Man erkennt, dass die Sub-Magnete 85 in der Mitte jeweils parallel zu dem gewünschten Verlauf der magnetischen Feldlinien stehen, aber auch nur dort. Je weiter ein Submagnet 85 zur Seite hineingesetzt wird, desto mehr wird er verdreht eingesetzt, wie sich aus der Lage des die Richtung 87 der Magnetisierung anzeigenden Pfeils ohne weite res ableiten lässt. Es ist insbesondere diese spezielle verdrehte Anordnung der Sub-Magnete 85, die ein weitgehend homo genes gerichtetes statisches magnetisches Feld in einem Innen raum 80 hervorrufen.

Durch das Betätigen des Magnetrings 8, insbesondere mittels einer manuellen Betätigungseinrichtung 75, wird der Magnetring 8 mitsamt seinen Sub-Magneten 85 in eine Drehbewegung in der Ringebene gebracht, wodurch sich entsprechend auch das gerichtete statische Magnetfeld 88 mitdreht. Die manuelle Betätigungseinrichtung 75 kann im einfachsten Fall als Handgriff 75 oder eine griffgünstige Beschichtung der Mantelfläche des Magnetrings 8 ausgeführt sein, es kann ggf. aber auch eine Mechanisierung mittels eines Servoantriebs (nicht dargestellt) vor gesehen sein. Bezogen auf einen Permanentmagneten als Antriebselement 3 kann so durch langsames Rotieren des Magnetrings 8 mit seinem statischen Magnetfeld 88 die Winkellage des An triebs der Verstelleinrichtung 2 und der aktuell erreichte Verstellweg einfach und sicher bestimmt werden. Somit ermöglicht die Antriebseinheit 7 schon bereits mit der manuellen Verstellung des Magnetrings 8 mittels des Betätigungselements 75 eine einfache aber dennoch präzise Steuerung und Kontrolle der tatsächlichen Position des Permanentmagneten, woraus sich wiederum die aktuelle Winkelstellung des Antriebs der Verstelleinrichtung 2 ergibt. Wegen der Stärke des in dem Hohlraum 80 des Magnetrings 8 erzeugten Magnetfelds und der Auslegung als ein statisches, d.h. gleichbleibendes starkes (wenn auch ggf. langsam rotierendes) Magnetfeld kommt dank dieser Anordnung ein die Positionierungsgenauigkeit verschlechternder Schlupf praktisch nicht vor. Es kann dem Benutzer diesbezüglich die Erfassung des erreichten Verstellwegs erleichtert werden, indem optional an der Antriebseinheit 7 ein Zählwerk 66 angeordnet ist, welches die Anzahl der Umdrehungen des Magnetrings 8 in der Antriebseinheit 7 überwacht und dem Benutzer anzeigt. Wegen der präzisen Übertragung der Drehbewegung des Magnetrings 8 auf den Permanentmagneten im Antrieb der Implantatkomponente 1 ist damit ein eindeutiges Maß für die von dem Verstelleinrichtung 2 der Implantatkomponente 1 bewirkte Verstellstrecke gegeben.

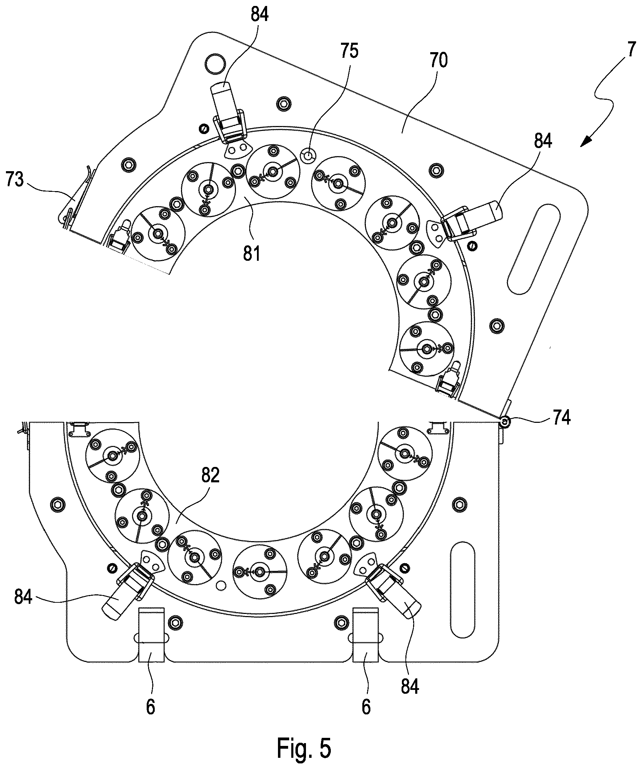

Ein Beispiel für eine konstruktive Ausführung der Antriebsein heit 7 mit Magnetring 8 wird nachfolgend unter Bezugnahme auf die Figuren 4 und 5 anhand einer ersten beispielhaften Ausführungsform erläutert. Die in ihrer Gesamtheit mit der Bezugsziffer 7 versehene Antriebseinheit umfasst einen Träger 70, in dem der Magnetring 8 drehbar gelagert ist. Hierbei fungiert die Innenseite der kreisförmigen Ausnehmung als Führung 78 für den Magnetring 8. Ferner sind im Träger 70 zwei Eingriffe 75' ausgeformt, die als Handgriffe zum Tragen vorgesehen sind. Sie ermöglichen ein problemloses Bewegen und Positionieren der Antriebseinheit 7. Im unteren Bereich der Antriebseinheit 7 sind zwei Standfüße 6 vorgesehen. Sie dienen dazu, die Antriebseinheit 7 in aufgerichteter Position kippsicher zu halten, so dass die Extremität (Bein 9) mit der Implantatkomponente 1 durch den Innenraum 80 des Magnetrings 8 geführt werden kann.

Der Magnetring 8 ist in zwei Segmente 81, 82 unterteilt, die jeweils als Halbkreisbogen ausgeführt sind. Der Magnetring 8 ist mit seinem Außenumfang über eine an einer Innenseite einer komplementär geformten kreisförmigen Ausnehmung im Träger 70 gelagert.

Der Träger 70 ist in eine obere und untere Hälfte geteilt entlang einer horizontal verlaufenden Teilungslinie. Für diese Teilungslinie sind an einer Lateralseite des Trägers 70 ein Schnallenverschluss 73 und an seiner gegenüberliegenden Late ralseite ein Scharnier 74 vorgesehen. Durch Öffnen des Schnallenverschlusses 73 kann die obere Hälfte des Trägers 70 nach oben hin aufgeklappt werden, und zwar mit dem Scharnier 74 als Drehpunkt. Beim Aufklappen verbleibt das Segment 81 des Magnetrings 8 an der oberen Hälfte und das Segment 82 des Magnetrings 8 an der unteren Hälfte des Trägers 70. Um ein Her ausfallen bzw. Verdrehen der Segmente 81, 82 zu vermeiden, sind zur Arretierung des Magnetrings 8 an dem Träger 70 insgesamt vier Arretierverschlüsse 84 vorgesehen, je zwei für eines der Segmente 81, 82. Im geschlossenen Zustand sind die beiden Segmente 81, 82 miteinander verbunden durch Spannverschlüsse 83'. Sind sie geschlossen, bilden die beiden Segmente 81 und 82 den einheitlichen Magnetring 8.

Der Magnetring 8 umfasst eine Vielzahl (bei den dargestellten Ausführungsbeispielen 14) von zylindrischen Sub-Magneten 85 aus Seltenerd-Material. Zu deren Halterung an dem Magnetring 8 sind passende Aufnahmen am Magnetring 8 vorgesehen. In diese werden die zylindrischen Sub-Magnete 85 eingesetzt und verdreh- sowie herausfallsicher befestigt. Die Verdrehsicherung ist bedeutend, da die einzelnen Sub-Magnete 85 mit genau definierter Winkel-orientierung eingesetzt und gehaltert sind. Die Winkelorientierung dient zu einer bestimmten Ausrichtung des durch den jeweiligen Sub-Magnets 85 erzeugten magnetischen Felds und ist durch Pfeile in Figur 4 und 5 visualisiert (vgl. zur Bedeutung der Pfeile auch die obige Erläuterung zu Figur 2 und 3b). Man erkennt, dass die Winkelorientierung der einzelnen Sub-Magnete 85 stets unterschiedlich von den benachbarten ist. Die Ausrichtung ist planvoll so gewählt, dass sich das Magnetfeld auf den Innenraum 80 konzentriert (der Außenraum des Magnetrings 8 ist im wesentlichen feldfrei), und so im Innenraum 80 ein im Wesentlichen homogenes, gerichtetes statisches Magnetfeld entsteht (vgl. Figur 2 und die obige Erläuterung dazu).

Durch insbesondere manuelles Verdrehen des Magnetrings 8 kann das so erzeugte statische Magnetfeld 88 rotiert werden, wobei ein sich im Innenraum 80 unter der Wirkung des Magnetfelds befindender Permanentmagnet entsprechend synchron mitdreht. Ein solcher Permanentmagnet wird als Antriebselement für die Implantatkomponente verwendet, wie später erläutert werden wird.

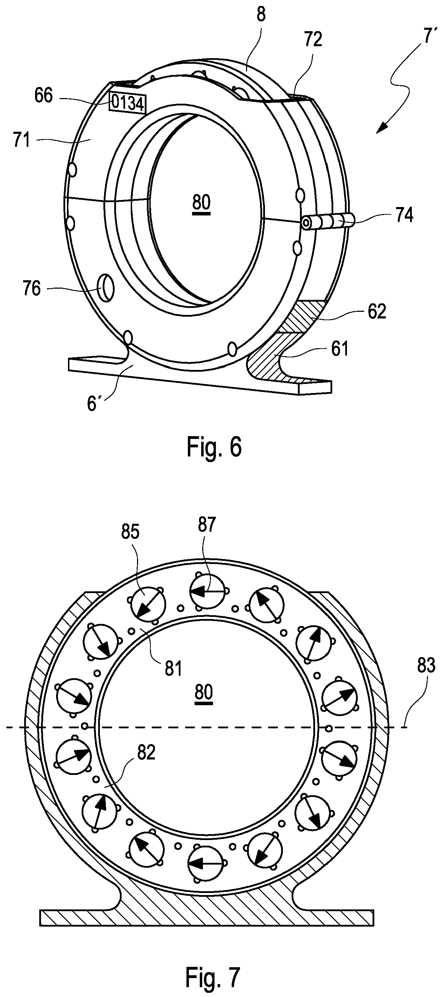

Eine zweite Ausführungsform für eine Antriebseinheit 7' ist in den Figuren 6 bis 11 dargestellt. Sie unterscheidet sich hin sichtlich ihrer Formgestaltung, ist funktional aber im Wesentlichen ähnlich zu der vorstehend beschriebenen ersten Ausführungsform. Für gleiche oder gleichartige Teile sind dieselben Bezugsziffern verwendet, wobei zur Vermeidung von unnötigen Wiederholungen auf obige Erläuterung verwiesen wird, die entsprechend sinngemäß anzuwenden ist.

Die zweite Ausführungsform der Antriebseinheit 7' weist statt des Trägers 70 ein den Magnetring umgebendes Gehäuse 71 auf.

Es umschließt den Magnetring 8 im Wesentlichen vollständig, mit Ausnahme einer im oberen Bereich des Gehäuses 71 großflächigen Öffnung 72. Sie ist so bemessen, um einen sich über mindestens 30°, mithin also ein Zwölftel des Umfangs des Magnetrings 8 erstreckenden Zugang zu ermöglichen, sodass der Benutzer den Magnetring 8 manuell verdrehen kann, insbesondere an der zur manuellen Betätigung ausgebildeten (mit Gripbeschichtung versehenen) Mantelfläche des Magnetrings 8. Es sei angemerkt, dass ein manueller Antrieb jedoch nicht zwingend ist. Für ein eventuelles Anflanschen eines Antriebsmotors ist eine Kupplungsöffnung 76 im Gehäuse 71 vorgesehen. Hier kann ein Antriebsmotor (nicht dargestellt) angeordnet werden, der auf den Magnetring 8 einwirkt und diesen in Drehung versetzt.

Es sei angemerkt, dass ein solch motorischer Antrieb ggf. auch bei der ersten Ausführungsform vorgesehen sein kann.

Das Gehäuse 71 ist vorzugsweise aus weichmagnetischen Material, beispielsweise hochpermeable Eisen-, Nickel- und/oder Kobaltlegierungen, um so den Außenraum vor dem magnetischen (Rest-)Feld des Magnetrings 8 abzuschirmen. Zweckmäßig sind insbesondere niedrig legierte Bau- und Automatenstähle oder Elektrobleche .

Vorzugsweise ist zumindest das Gehäuse 71 im Bereich der Mantelfläche des Magnetrings 8 schirmend ausgeführt. Zweckmäßigerweise ist hierbei das Gehäuse 71 so bemessen, dass es im Bereich der Schirmung (z. B. Mantelfläche) einen Abstand von dem Magnetring 8 aufweist, der mindestens so groß ist wie Durchmesser/Höhe der einzelnen Sub-Magnete 85, vorzugsweise ein Mehrfaches davon. Damit kann eine gute Schirmwirkung auch mit verhältnismäßig geringen Materialstärken (typischerweise 0,5 mm oder weniger) und somit wenig Gewicht erreicht werden. Soll optional auch eine Schirmung im Bereich der Stirnseiten des Magnetrings 8 bewirkt werden, so wäre entweder das Gehäuse 71 mit recht großer Tiefe auszuführen, um den o.g. Abstand der stirnseitigen Gehäusewand von mindestens dem Durchmesser/Höhe der einzelnen Sub-Magnete 85 zu erreichen, oder die Materialstärke der stirnseitigen Gehäusewand wäre erheblich höher zu wählen als im Bereich der Mantelfläche, meist mehr als doppelt oder gar dreimal so dick (d. h. typischerweise größer als 1 mm) .

Das Gehäuse 71 ist ferner versehen mit einer richtungsorientierten Aufnahme für eine Extremität, beispielsweise einem Bein. Hier soll jedoch nicht die mit der Implantatkomponente 1 versehene Extremität aufgenommen sein, sondern deren kontralaterales Gegenstück, also das andere Bein. Das Gehäuse 71 ist dazu, wie in Figur 6 exemplarisch dargestellt, im Bereich sei nes Standfußes 6' mit einer im Querschnitt im Wesentlichen V-förmigen Ausnehmung versehen, die eine Aufnahme 61 bildet. Sie ist in dem dargestellten Ausführungsbeispiel so bemessen, dass im Bereich der Aufnahme 61 der Unterschenkel des anderen Beins (nicht dargestellt) aufgenommen werden kann oder alternativ/zusätzlich so viel größer bemessen, dass im Bereich der Aufnahme 62 der Oberschenkel des anderen Beins (nicht dargestellt) aufgenommen werden kann. Die Aufnahme 61, 62 ist zweckmäßigerweise so geformt, dass sie die Extremität nur in einer definierten Position aufnimmt. Wird dementsprechend der Unter- oder Oberschenkel des besagten anderen Beins in der Aufnahme 61, 62 platziert, so ist die Antriebseinheit 7' sozusagen automatisch korrekt ausgerichtet zur Verstellung der einzustellenden Implantatkomponente 1 (für andere Extremitäten, wie Arme, gilt entsprechendes). Dies vereinfacht für den Benutzer die korrekte Anwendung bzw. Positionierung der erfindungsgemäßen Antriebseinheit 7'. Es sei angemerkt, dass die Aufnahmen 61, 62 auch als individuell angepasste Ausformungen ausgeführt sein können.

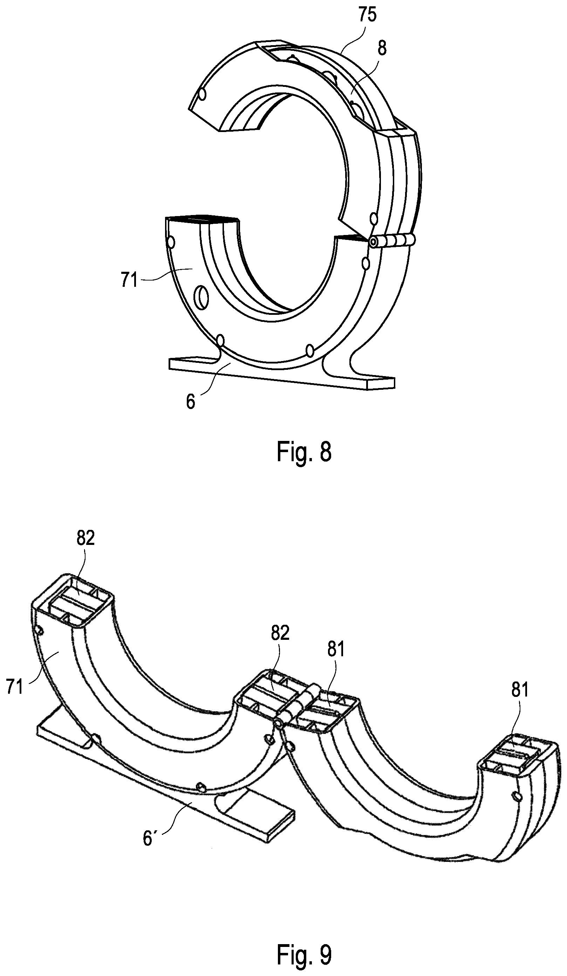

Wie auch schon bei der ersten Ausführungsform ist der Magnet ring 8 versehen mit einer Vielzahl von Sub-Magneten 85, die in der Teilschnittdarstellung in Figur 7 dargestellt sind. Man erkennt auch hier wieder die durch die Pfeile 87 visualisierte Ausrichtung der Sub-Magnete 85, um das konzentrierte gerichtete statische Magnetfeld im Innenraum 80 zu bilden. Auch hier ist wieder der Magnetring 8 in zwei Segmente 81, 82 unterteilt, welche zusammen mit der oberen Hälfte des Gehäuses 71 aufgeklappt werden können (siehe Figur 8 und 9). Wie bei der ersten Ausführungsform ist zu diesem Zweck an einer Seite ein Scharnier 74 vorgesehen. Figur 9 zeigt die zweite Ausführungsform mit vollständig aufgeklapptem Gehäuse 71. Man kann an den Trennflächen gut die jeweiligen Enden der Segmente 81, 82 erkennen, die im zusammengeklapptem Zustand (siehe Figur 7) in der Trennebene 83 liegen.

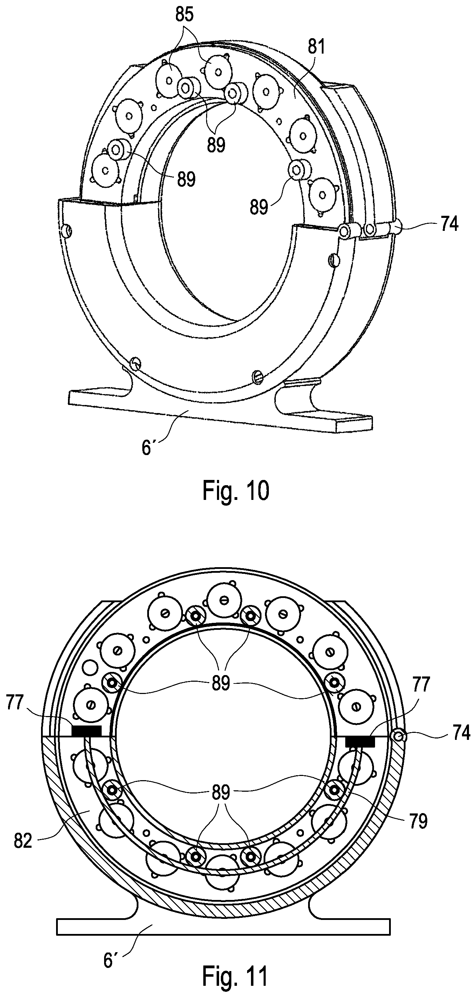

Bei der in Figur 10 gezeigten Darstellung ist ein Teil des oberen Bereichs des Gehäuses 71 entfernt. Man erkennt hierbei zusätzlich zu dem oberen Segment 81 des Magnetrings 8 deutlich dessen Submagnete 85, wie vorstehend beschrieben. Ferner er kennt man eine Mehrzahl von Führungsrollen 89 an dem Magnet ring 8, die mit einer im unteren Bereich des Gehäuses 71 angeordneten bogenförmigen Führungsschiene 79 Zusammenwirken (siehe Figur 11). Auf diese Weise wird eine sichere Zwangsführung des Magnetrings 8 in dem Gehäuse 71 erreicht. Es genügt, dass sich die bogenförmige Führungsschienen 79 über die untere Gehäusehälfte erstreckt, da sich - dank der Verbindung der Segmente 81, 82 im zusammengeklapptem Normalzustand - auf diese Weise eine vollständige Führung des Magnetrings 8 ergibt. Im aufgeklappten Zustand gilt dies zwar für das obere Segment 81 nicht, jedoch ist dies dank seiner Bogenform und eines Sperrbolzens 84' in der gleichfalls bogenförmigen oberen Hälfte des Gehäuses 71 insoweit gesichert, als dass es nicht herausfallen kann. Beim Zuklappen des Gehäuses 71 und nachfolgender gegenseitiger Verriegelung der Segmente 81, 82 wird der Magnetring 8 wieder geschlossen, und die Antriebseinrichtung 7' ist dann wieder ohne weiteres Zutun einsatzbereit.

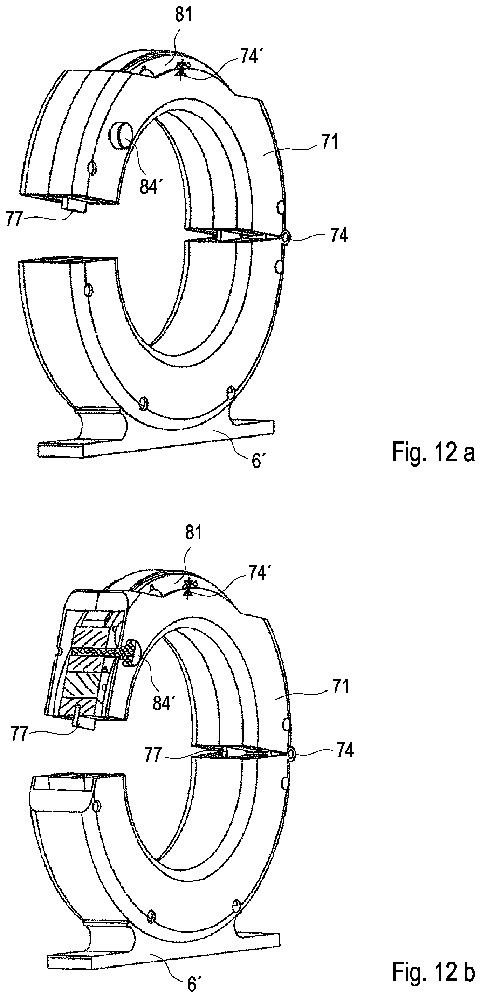

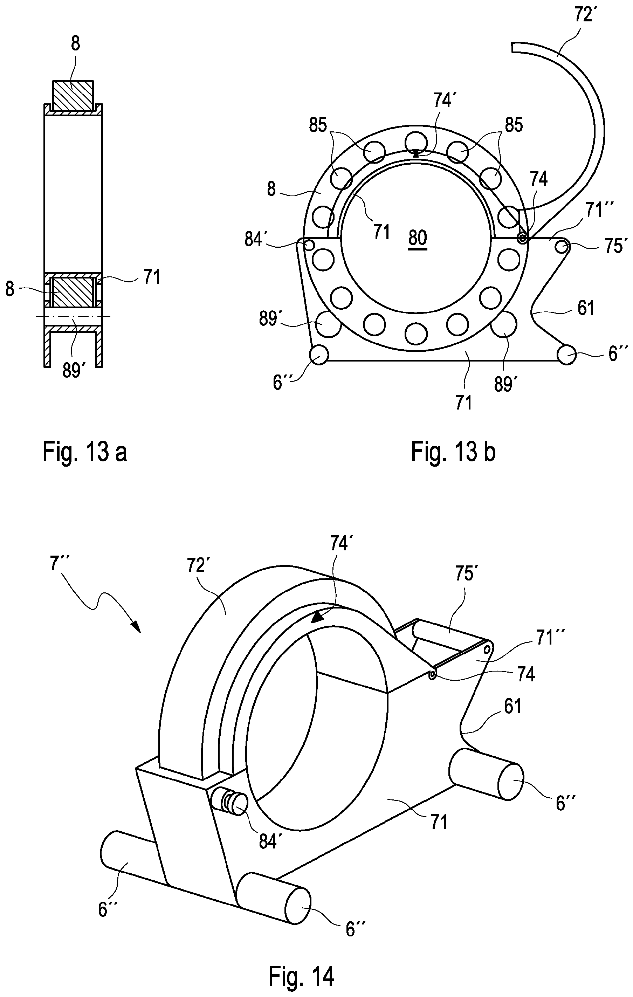

Um eine korrekte relative Positionierung der beiden Segmente 81, 82 im geschlossenen (zusammengeklappten) Zustand zu gewährleisten, sind Führungselemente 77 vorgesehen. Sie sind klinkenartig ausgeführt und an den beiden Enden der Führungsschiene 79 im Bereich der Trennebene 83 an den Segmenten 81, 82 angeordnet (s. Fig. 12 und 13). Sie sind Teil einer Federrastmechanik, welche die Segmente 81, 82 zusammen hält. Es ergibt sich so im geschlossenen Zustand eine wirksame Führung und Sicherung für den Magnetring 8, und zwar mittels der an der Führungsschiene 79 geführten Führungsrollen 89 und den Führungselementen 77 an den Enden der Führungsschiene 79.

Zur Arretierung in der gesicherten Position ist ein Sperrbolzen 84' vorgesehen, der in entsprechende Aufnahmeöffnungen im oberen Bereich des Gehäuses 71 und im oberen Segment 81 des Magnetrings 8 steckbar ist (s. Fig. 12 a, b). Die Position, in der der Magnetring 8 stehen muss zum Einstecken des Sperrbolzen 84', ist diejenige, in der die Trennebene 83 zwischen den Segmenten 81, 82 fluchtet mit dem Scharnier 74 des Gehäuses 71 - nur in dieser Grundposition ist ein Aufklappen möglich. Die korrekte Stellung des Magnetrings 8 in dieser Grundposition ist angezeigt durch eine im Bereich der Gehäuseöffnung 72 an geordnete Referenzmarkierung 74', deren eine Hälfte am Gehäuse 71 und die andere Hälfte am Magnetring 8 angebracht ist (bspw. als Doppelpfeilmarkierung) - stehen beide übereinander, ist die Grundposition erreicht.

In dieser Grundposition fluchten die Aufnahmeöffnungen in Gehäuse 71 und Segment 81 des Magnetrings 8, und der Sperrbolzen 84' kann eingesteckt werden. Damit ergibt sich eine Zwangsführung, mittels der der Magnetring 8 arretiert wird wobei zu gleich mittels mechanischer Kombination die Federrastmechanik entriegelt wird, so dass die Segmente 81, 82 entkoppelt werden und der Magnetring 8 aufgeklappt werden kann. Durch den Sperr bolzen 84' ist hierbei auch beim Aufklappen bzw. im aufgeklappten Zustand das obere Segment 81 des Magnetrings 8 gegen über unerwünschter Bewegung gesichert.

Eine dritte Ausführungsform für eine Antriebseinheit 7" ist in Fig. 13a, b und 14 dargestellt. Sie basiert auf der zweiten Ausführungsform, weist im Gegensatz zu dieser aber ein anders gestaltetes Gehäuse 71 auf. Es weist im oberen Bereich einen äußeren Schutzdeckel 72' auf, der klappbar am Scharnier 74 gelagert ist und im geöffneten Zustand den oberen Bereich des Magnetrings 8 freilegt. Im Bereich des Scharniers 74 ist ein Ausleger 71" am Gehäuse 71 vorgesehen. Er trägt zum einen den Handgriff 75' für den Transport der Antriebseinheit 7" und bildet zum anderen auf diese Weise einen Anschlag für den Schutzdeckel 72', um so eine Öffnungsbegrenzung zu erreichen.

Der Ausleger 71" ist ferner so ausgeformt, dass er mit seiner Seitenkontur eine Aufnahme 61 für die kontralaterale Extremität (das andere Bein) bietet. Im Übrigen stimmt deren Funktionalität mit derjenigen der Aufnahme 61 bei der zweiten Ausführungsform überein, so dass zur Vermeidung von Wiederholungen darauf verwiesen wird. Das gilt entsprechend auch für die anderen Komponenten der dritten Ausführungsform, die gleiche oder entsprechende Bezugsnummern wie die korrespondierenden Komponenten der zweiten Ausführungsform tragen.

Als Besonderheit weist die dritte Ausführungsform ferner ein Gegenstück zu dem äußeren Schutzdeckel 72' auf. Es ist innenseitig des Magnetrings 8 ein innerer Schutzdeckel 71' vorgesehen, der den Innenraum 80 vom Magnetring 8 separiert. Er fungiert nicht nur als mechanische Abdeckung, sondern dient auch zur Abschirmung des Umgebungsbereichs, insbesondere benachbarter Bereiche der aufzunehmenden Extremität (insbesondere Bein 9), vor unnötiger Belastung durch magnetische Felder.

Der Magnetring 8 ist gelagert auf gehäusefest angeordneten Führungsrollen 89', auf denen der Magnetring 8 in seinem unteren Bereich mit seiner Mantelfläche aufliegt. Dies ermögliche einen kompakten Aufbau mit verhältnismäßig tief liegendem Schwerpunkt. Der Gefahr eines ungewollten Umkippens der Antriebseinheit 7" kann damit begegnet werden. Optional sind die Führungsrollen 89' an ihren Stirnseiten mit vergrößertem Durchmesser ausgeführt, so dass ein Bund entsteht und eine Seitenführung für den Magnetring 8 erreicht ist. Es können auch zusätzliche Führungsrollen 89' insbesondere im oberen Be reich des Gehäuses vorgesehen sein.

Ferner weist bei dieser dritten Ausführungsform das Gehäuse 71 doppelt ausgeführte Standfüße 6" auf. Sie geben der Antriebseinheit 7" eine breitere Aufstandsfläche und erhöhen so weiter die Sicherheit gegenüber Umkippen. Im Übrigen wird zur Vermeidung von Wiederholungen auf die vorstehende Beschreibung zu der zweiten Ausführungsform verwiesen.

Am Magnetring 8 bzw. den umgebenden Gehäuse 71 und seinen Komponenten 71', 72' können magnetische Schirmungen vorgesehen sein, vorzugsweise aus magnetischen gut leitfähigem (hochpermeablen) Material, insbesondere weichmagnetische (ferromagnetische) Werkstoffe wie Eisen-, Nickel- und Kobaltlegierungen. Der Übersichtlichkeit halber sind verschiedene Schirmungen nachfolgend erläutert unter Bezugnahme auf Fig. 15 anhand der ersten Ausführungsform, wobei für die anderen Ausführungsformen entsprechendes gilt. Es kann eine Außenschirmung 67 vorgesehen sein in verhältnismäßig großen Abstand, worunter ein Vielfaches des Durchmessers bzw. der Höhe der Submagnete 85 verstanden wird. Bei einer solchen beabstandeten Schirmung sind Materialstärken des Schirmungsmaterials von nur wenigen Zehntelmillimetern ausreichend und von Vorteil.

Zur Schirmung am Magnetrad 8 ist zweckmäßigerweise eine direkt benachbarte Mantelschirmung 68 vorgesehen. Typischerweise ist hier der Abstand der Schirmung etwa gleich dem Durchmesser bzw. der Höhe der Submagnete 85, so dass Materialstärken von mindestens einem halben Millimeter, vorzugsweise bis zu einem Millimeter zweckmäßig sind. Eine solche Mantelschirmung kann auch innenseitig zweckmäßig sein, beispielsweise an dem inneren Schutzdeckel 71' der dritten Ausführungsform zum Schutz umliegender Bereiche des betroffenen Beins 9.

Weiter kann eine Schirmung auch an den Stirnflächen des Magnetrads vorgesehen sein, als Nahfeldschirmung 69. Der Abstand ist hierbei typischerweise kleiner als der Durchmesser bzw. die Höhe der Submagnete 85, so dass es zu einer starken magnetischen Aussteuerung des Weicheisens kommt. Erforderlich sind daher recht große Materialstärken von mindestens einem, meist mehreren Millimetern.

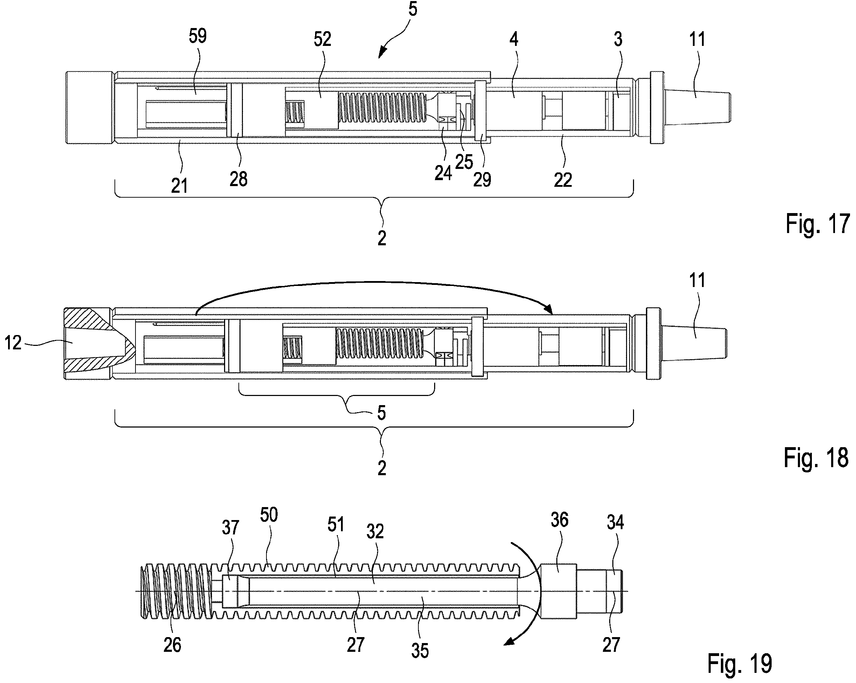

Ein Ausführungsbeispiel für eine Implantatkomponente 1 ist in den Fig. 17 bis 19 dargestellt. Figur 17 zeigt ein Teilschnitt durch die Implantatkomponente 1 mit ihrer Verstelleinrichtung 2. Die Implantatkomponente 1 ist in dem dargestellten Ausführungsbeispiel schaftartig geformt und bildet somit Teil eines Prothesenschafts, insbesondere für ein modulares Prothesensystem.

Sie weist an ihren beiden Enden jeweils ein Kopplungselement an benachbarte Prothesenmodule eines vorzugsweise standardisierten Prothesensystems auf, beispielsweise einen weiteren Schaft oder einen Anschluss an eine Gelenkkomponente. So weist die Implantatkomponente 1 an ihrem in Fig. 17 rechts dargestellten Ende einen männlichen Konus 11 als Kopplungselement und an ihrem in der Darstellung links dargestellten Ende ein als weiblichen Konus 12 ausgeführtes Kopplungselement zum Anschluss weiterer Prothesenmodule (nicht dargestellt) auf; es versteht sich, dass auch andere Verbindungsarten als Konusverbinder vorgesehen sein können.

Der eigentliche Schaftkörper zwischen den Kopplungselementen 11, 12 ist ausgeführt mit einem Außenrohr 21 und einem darin längsverschieblich geführten Innenrohr 22. Sie sind Teil einer Verstelleinrichtung 2 und wirken teleskopartig zusammen, um so die Einstellung unterschiedlicher Längen für die Implantatkomponente 1 zu ermöglichen. Das Außenrohr 21 und das Innenrohr 22 sind über zwei Gleitführungen miteinander verbunden, von denen die eine Gleitführung 28 am freien Ende des Innenrohres 22 und beweglich im Außenrohr 21 angeordnet ist, während die andere Gleitführung 29 fest am Ende des Außenrohrs 21 im Be reich des Übergangs zum Innenrohr 22 angeordnet ist. Ferner ist eine Führungsnut innenwandig am Außenrohr 21 angeordnet, um eine gegenseitige Verdrehung des Innenrohrs 22 zum Außenrohr 21 zu vermeiden. Als Lager für die Längsverstellung dient ein ebenfalls am Ende des Außenrohrs 21 angeordnetes Axiallager 25, dem ein Dichtringpaar 24 zugeordnet ist, um das Innere des Außenrohrs 21 abzuschließen gegenüber umgebenden Körpergewebe bzw. -fluiden.

Die Verstelleinrichtung 2 zur Einstellung der Länge der Implantatkomponente 1 umfasst weiter ein Antriebselement 3, welches einen drehbeweglich um eine Mittelachse des Innenrohrs angeordneten Permanentmagneten aufweist. Er ist mit einer solchen Ausrichtung angeordnet, ausgebildet, dass seine Polarisierungsrichtung (Richtung vom Nordpol zum Südpol) orthogonal zu der Mittelachse steht. Zweckmäßigerweise ist hierfür ebenfalls ein Sub-Magnet 85, wie vorstehend beschrieben, verwendet. Dem Antriebselement 3 nachgeordnet ist ein Getriebe 4 als ein Reduktionsgetriebe. In dem dargestellten Ausführungsbei spiel ist es als zweistufiges Planetengetriebe ausgeführt, dass eine Untersetzung von etwa 1 zu 20 aufweist.

Das Getriebe 4 wirkt auf einen Spindeltrieb 5, der eine Drehbewegung des Getriebes 4 umsetzt in eine Linearbewegung zur Längenverstellung. Zur Drehmomentübertragung zwischen Getriebe 4 und Spindeltrieb 5 ist ein Biegestab 35 vorgesehen. Das Ge triebe 4 wirkt auf ein getriebenes Ende 34 des Biegestabs 35, der koaxial zu der Mittelachse 27 des Innenrohrs 22 angeordnet ist. Der Biegestab 35 weist weiter einen Bund 36 auf, der im Bereich der Dichtringe 24 angeordnet ist, sowie daran anschließend einen langgestreckten eingeschnürten Schaftbereich 32 aufweist, der am freien Ende des Biegestabs 35 an einem Kopplungsstück 37 endet. Der langgestreckte eingeschnürte Schaftbereich 32 ist eingesteckt in eine einseitig offene zentrische Bohrung 51 der Antriebsspindel 50, wobei das Koppelstück 37 am Grund der zentrischen Bohrung 51 mit der Antriebsspindel 50 verbunden ist. Die von dem Biegestab 35 in Drehbewegung versetzte Antriebsspindel 50 rotiert um eine Mittelachse 26 des Außenrohrs 21 und bewirkt so eine Längsbewegung eines auf der Antriebsspindel 50 angeordneten Verstellschlittens 52, der fest mit dem Innenrohr 22 fest verbunden ist. So wird im Ergebnis durch eine Drehung des Antriebsmagneten 3 - über das Reduktionsgetriebe 4 und den Spindeltrieb 5 - eine Längsverstellung der Implantatkomponente 1 bewirkt.

Es wird nun Bezug genommen auf Figur 18 und 19. Im Betrieb kommt es unter Last zu Biegebeanspruchung in der Verstelleinrichtung 2. Eine durch die Längenverstellung der Implantatkomponente 1 entstehende wesentliche Belastung ist eine Knicklast, welche die im Wesentlichen schaftartige Implantatkomponente 1 auf Durchbiegung beansprucht. Diese Beanspruchung ist durch einen langgestreckten Pfeil in Figur 18 symbolisiert. Als Folge dieser Beanspruchung können Winkelfehler entstehen zwischen der Mittelachse 26 des Außenrohrs 21 und der Mittelachse 27 des Innenrohrs 22, d. h. unter Last fluchten die bei den Mittelachsen 26, 27 nicht mehr, sondern weisen einen Fluchtungsfehlerwinkel von einigen Grad auf.

Die empfindliche Drehmomentübertragung von dem Antriebsmagneten 3 auf den Spindeltrieb 5 kann dadurch erheblich gestört werden, wodurch es zu einem unerwünschten Blockieren kommen kann. Abhilfe bietet hier erfindungsgemäß der Biegestab 35, welcher dank seiner Verbiegung den Fluchtungsfehler aufnehmen kann (symbolisiert durch die Pfeildarstellung in Fig. 19). Er ermöglicht die Kompensation von Fluchtungsfehlern im Bereich von bis zu +/- 5 Grad, vorzugsweise bis zu +/- 2 Grad. Er vergrößert somit die Toleranz des Antriebsstrangs gegenüber Biegebeanspruchung und erhöht somit die Belastbarkeit sowie Zuverlässigkeit der Verstelleinrichtung 2 der Implantatkomponente 1.

Verstellt wird die Implantatkomponente 1 mit ihrer Verstellungseinrichtung 2, indem die Extremität (Bein 9) mit der Implantatkomponente 1 so in dem Innenraum 80 der Antriebseinheit 7, 7' platziert wird, dass die Verstelleinrichtung 2 mit ihrem Antriebsmagneten 3 in der Ebene des Magnetrings 8 liegt. Durch Betätigen der Antriebseinheit 7, 7', und zwar in dem der Magnetring 8 in der Regel von Hand verdreht wird, wirkt das mit dem Magnetring 8 synchron mitdrehende Magnetfeld 88 auf den Antriebsmagneten 3, der dann ebenfalls entsprechend synchron mitgedreht wird. Durch das statische Magnetfeld, das anders als ein typisches elektromagnetisch erzeugtes Feld (insbesondere einer Elektromotorwicklung) nicht hinsichtlich seiner Stärke moduliert ist, sondern eine konstante Stärke aufweist, wird ein Schlupf zwischen Antriebsmagnet 3 und Magnetring 8 zuverlässig vermieden. Somit ist die Winkelposition wie auch die Zahl der Umdrehungen des Magnetrings 8 gleich derjenigen des Antriebsmagneten 3. Es kann so auf einfache Weise eine präzise Längenverstellung durchgeführt werden. Die Anzahl der zur Verstellung vorgenommen Umdrehungen (und damit die so erreichte Verstellstrecke) wird hierbei auf dem Zählwerk 66 für den Benutzer angezeigt.

## Patentansprüche

1. Implantatkomponente mit einer Verstelleinrichtung (2) für eine implantierbare Prothese, die zur Befestigung an einem zu verlängernden Knochen (90) ausgebildet ist, umfassend zwei gegeneinander verschiebliche Befestigungsteile (21, 22), die mittels der Verstelleinrichtung (2) relativ zueinander verschoben werden zur Längenexpansion, wobei die Verstelleinrichtung (2) ein Antriebselement (3) mit einem drehbeweglich angeordneten Permanentmagnet umfasst, der über ein Getriebe (4) auf einen Spindeltrieb (5) wirkt zum Umwandeln einer Drehbewegung des Permanentmagnets in eine distrahierende Längsbewegung, wobei das Antriebselement (3) zur Betätigung mittels eines Magnetfelds einer extrakorporalen Antriebseinheit (7, 7', 7") ausgebildet ist, **dadurch gekennzeichnet, dass** zur Kraftübertragung von dem Antriebselement (3) auf den Spindeltrieb (5) ein Biegestab (35) vorgesehen ist.

2. Implantatkomponente mit Verstelleinrichtung nach Anspruch 1, **dadurch gekennzeichnet, dass** eine Spindel (50) des Spindeltriebs (5) eine einseitig offene zentrische Bohrung (51) aufweist, in die der Biegestab (35) eingesteckt ist.

3. Implantatkomponente mit Verstelleinrichtung nach Anspruch 2, **dadurch gekennzeichnet, dass** der Biegestab (35) an seinem freien, von dem Antriebselement (3) fernen Ende angekoppelt ist an ein Kopplungsstück (32) am Grund der zentrischen Bohrung, und der Biegestab (35) vorzugsweise einen eingeschnürten Schaft aufweist.

4. Implantatkomponente mit Verstelleinrichtung nach einem der Ansprüche 1 bis 3, **dadurch gekennzeichnet, dass** der Biegestab (35) eine Länge aufweist, die vorzugsweise mindestens ein Drittel, weiter vorzugsweise mindestens eine Hälfte, der Länge der Antriebsspindel (50) beträgt, und insbesondere dazu ausgebildet ist, einen Winkelversatz von bis zu +/- 5 Grad aufzunehmen.

5. Implantatkomponente mit Verstelleinrichtung nach einem der Ansprüche 1 bis 4, **dadurch gekennzeichnet, dass** der Biegestab (35) aus einer Titanlegierung TiA16V4 besteht.

6. Extrakorporal längenverstellbares Implantatsystem umfassend
- eine Implantatkomponente (1) nach einem der vorgehenden Ansprüche; und
- eine Extrakorporale Antriebseinheit (7, 7', 7") zur Betätigung des Antriebselements (3) mittels eines Magnetfelds, wobei die extrakorporale Antriebseinheit (7, 7', 7") einen drehbar gelagerten extrakorporalen Magnetring (8) mit einem Innenraum (80), der zur Aufnahme des zu verlängernden Knochens (90) mit der Verstelleinrichtung (2) ausgebildet ist, und eine Betätigungseinrichtung (75) aufweist, die dazu ausgebildet ist, den extrakorporalen Magnetring (8) in seiner Ringebene zu rotieren, wobei der extrakorporale Magnetring (8) in Permanentmagnetbauweise ausgeführt ist und in seinem Innenraum ein gerichtetes statisches magnetisches Feld aufweist, das ringfest ist, und der extrakorporale Magnetring (8) mechanisch von der Betätigungseinrichtung (75) rotiert ist.

7. Implantatsystem nach Anspruch 6, **dadurch gekennzeichnet, dass**
- das magnetische Feld auf den Innenraum (80) konzentriert ist, und/oder
- das gerichtete statische magnetische Feld im zentralen Innenraum unidirektional und/oder homogen ist.

8. Implantatsystem nach Anspruch 6 oder Anspruch 7 **dadurch gekennzeichnet, dass** der Magnetring teilbar ist in mindestens zwei Segmente (81, 82), um so einen öffenbaren und verschließbaren Zugang für ein mit der Implantatkomponente (1) versehenes Körperteil, insbesondere Bein oder Arm, zum Innenraum des Magnetrings (8) zu schaffen, wobei die Segmente (81, 82) in einer Öffnungsposition des Magnetrings (8) aufklappbar sind, wobei, vorzugsweise:
- die Segmente (81, 82) in einer Öffnungsposition des Magnetrings (8), vorzugsweise magnetkraftfrei, aufklappbar sind; und/oder
- eine Trennebene (83) zwischen den Segmenten so gewählt ist, dass sie sich parallel zu dem gerichteten statischen magnetischen Feld erstreckt; und/oder
- die Segmente mit Verbindungsmitteln verbunden sind, von denen mindestens eines (73) öffen- und wiederverschließbar und mindestens eines scharnierartig (74) klappbar ist; und/oder
- eine Arretiervorrichtung (84) vorgesehen ist, die den Magnetring (8) mit seinen Segmenten (81, 82) in einer Öffnungsposition verdrehsicher fixiert; und/oder
- am Magnetring (8) eine Sicherungseinrichtung vorgesehen, die insbesondere mittels eines Sperrbolzens (84') dazu ausgebildet ist, ein Aufklappen des Magnetrings (8) außerhalb der Öffnungsposition zu blockieren.

9. Implantatsystem nach einem der Ansprüche 6 bis 8 **dadurch gekennzeichnet, dass** der Magnetring (8) gebildet ist mittels einer Vielzahl von vorzugsweise gleichartigen Sub-Magneten (85), wobei vorzugsweise die Sub-Magnete gleichartige magnetische Dipolkörper (86) sind, insbesondere gebildet aus Seltenerd-Magneten.

10. Implantatsystem nach Anspruch 9, **dadurch gekennzeichnet, dass** die Sub-Magnete (85) entlang des Magnetrings (8) regelmäßig mit verschiedener Orientierung (87) der Magnetisierung winkelfest eingesetzt sind.

11. Implantatsystem nach Anspruch 10, **dadurch gekennzeichnet, dass** die Sub-Magnete (85) so angeordnet sind, dass sich ihr magnetisches Streufeld entlang einer Trennebene (83) zwischen Segmenten (81, 82) des Magnetrings (8) jeweils maximal kompensiert, wobei die Sub-Magnete (85) vorzugsweise so angeordnet sind, dass ihr magnetisches Streufeld entlang einer Trennebene (83) zwischen Segmenten (81, 82) des Magnetrings (8) gleich Null ist.

12. Implantatsystem nach einem der Ansprüche 6 bis 11 **dadurch gekennzeichnet, dass** ein Gehäuse (71) für die Antriebseinheit (7, 7', 7") vorgesehen ist, das vorzugsweise mit einer oder mehreren Schirmungen (67, 68, 69) versehen ist und insbesondere den Magnetring (8) außenfeldfrei umgibt.

13. Implantatsystem nach einem der Ansprüche 6 bis 12 **dadurch gekennzeichnet, dass** die Antriebseinheit (7, 7', 7") mit einer Positionierungseinrichtung versehen ist, die auf den Magnetring (8) wirkt, insbesondere ein Umdrehungen des Magnetrings anzeigendes Zählwerk (66).

14. Implantatsystem nach einem der Ansprüche 6 bis 13 **dadurch gekennzeichnet, dass** die Antriebseinheit mit einem Standfuß (6) als Kippschutz versehen ist, der vor zugsweise mit einer richtungsorientierten Aufnahme (61, 62) für ein mit der Prothese versehenes Körperteil (9), insbesondere Arm oder Bein, versehen ist.

## Claims

1. An implant component with an adjusting device (2) for an implantable prosthesis, which is designed for fastening to a bone (90) to be extended, comprising two fastening parts (21, 22) which are displaceable relative to one another, which are displaced relative to one another by means of the adjusting device (2) for longitudinal expansion, wherein the adjusting device (2) comprises a drive element (3) with a permanent magnet which is arranged so as to be rotatable and which acts via a gear (4) on a spindle drive (5) for converting a rotary movement of the permanent magnet into a distracting longitudinal movement, wherein the drive element (3) is designed for actuation by means of a magnetic field of an extracorporeal drive unit (7, 7', 7"), **characterized in that** a bending rod (35) is provided for force transmission from the drive element (3) to the spindle drive (5).

2. The implant component with an adjusting device according to claim 1, **characterized in that** a spindle (50) of the spindle drive (5) has a central bore (51) which is open on one side and into which the bending rod (35) is inserted.

3. The implant component with an adjusting device according to claim 2, **characterized in that** the bending rod (35) is coupled at its free end remote from the drive element (3) to a coupling piece (32) at the base of the central bore and the bending rod (35) preferably has a constricted shank.

4. The implant component with an adjusting device according to any one of claims 1 to 3, **characterized in that** the bending rod (35) has a length which is preferably at least one third, more preferably at least one half, of the length of the drive spindle (50), and is designed in particular to accommodate an angular offset of up to +/- 5 degrees.

5. The implant component with an adjusting device according to any one of claims 1 to 4, **characterized in that** the bending rod (35) consists of a TiA16V4 titanium alloy.

6. An extracorporeally length-adjustable implant system, comprising
- an implant component (1) according to any one of the preceding claims; and
- an extracorporeal drive unit (7, 7', 7") for actuating the drive element (3) by means of a magnetic field, wherein the extracorporeal drive unit (7, 7', 7") has a rotatably mounted extracorporeal magnetic ring (8) with an interior space (80) which is designed to receive the bone (90) to be extended with the adjusting device (2), and an actuating device (75) which is designed to rotate the extracorporeal magnetic ring (8) in its ring plane, wherein the extracorporeal magnetic ring (8) is embodied in a permanent magnet design and has a directed static magnetic field in its interior space which is fixed in the ring, and the extracorporeal magnet ring (8) is rotated mechanically by the actuating device (75).

7. The implant system according to claim 6, **characterized in that**
- the magnetic field is concentrated on the interior space (80), and/or
- the directed static magnetic field in the central interior space is unidirectional and/or homogeneous.

8. The implant system according to claim 6 or claim 7, **characterized in that** the magnetic ring can be divided into at least two segments (81, 82) so as to create an openable and closable access for a body part provided with the implant component (1), in particular a leg or arm, to the interior space of the magnetic ring (8), wherein the segments (81, 82) can be folded open in an open position of the magnetic ring (8), wherein preferably:
- the segments (81, 82) can be folded open, preferably without magnetic force, in an open position of the magnetic ring (8); and/or
- a separation plane (83) between the segments is selected to extend parallel to the directed static magnetic field; and/or
- the segments are connected to connecting means, of which at least one (73) can be opened and closed again and at least one (74) can be folded in the manner of a hinge; and/or
- a locking device (84) is provided which fixes the magnetic ring (8) with its segments (81, 82) in an opening position in a rotationally secure manner; and/or
- a safety device is provided on the magnetic ring (8), which is designed, in particular by means of a locking bolt (84'), to prevent the magnetic ring (8) from folding open outside the opening position.

9. The implant system according to any one of claims 6 to 8, **characterized in that** the magnetic ring (8) is formed by means of a plurality of preferably identical sub-magnets (85), wherein preferably the sub-magnets are identical magnetic dipole bodies (86), in particular formed from rare earth magnets.

10. The implant system according to claim 9, **characterized in that** the sub-magnets (85) are inserted along the magnetic ring (8) in a regular manner with different orientation (87) of the magnetization in an angularly fixed manner.

11. The implant system according to claim 10, **characterized in that** the sub-magnets (85) are arranged so that their magnetic stray fields each maximally compensate for one another along a separating plane (83) between segments (81, 82) of the magnetic ring (8), wherein the sub-magnets (85) are preferably arranged so that their magnetic stray field is equal to zero along a separating plane (83) between segments (81, 82) of the magnetic ring (8).

12. The implant system according to any one of claims 6 to 11, **characterized in that** a housing (71) is provided for the drive unit (7, 7', 7"), which is preferably provided with one or more shieldings (67, 68, 69) and in particular surrounds the magnetic ring (8) free of external fields.

13. The implant system according to any one of claims 6 to 12, **characterized in that** the drive unit (7, 7', 7") is provided with a positioning device which acts on the magnetic ring (8), in particular a counter (66) indicating revolutions of the magnetic ring.

14. The implant system according to any one of claims 6 to 13, **characterized in that** the drive unit is provided with a standing foot (6) as tilting protection, which is preferably provided with a directionally-oriented receptacle (61, 62) for a body part (9) provided with the prosthesis, in particular an arm or a leg.

## Revendications

1. Composant d'implant avec un dispositif de réglage (2) pour une prothèse implantable, qui est conçu pour être fixé à un os (90) à prolonger, comprenant deux parties de fixation (21, 22) qui sont déplaçables l'une par rapport à l'autre, lesquelles sont déplacées l'une par rapport à l'autre au moyen du dispositif de réglage (2) pour un prolongement longitudinal, dans lequel le dispositif de réglage (2) comprend un élément d'entraînement (3) avec un aimant permanent qui est agencé de manière à être rotatif et qui agit via un engrenage (4) sur un entraînement à broche (5) pour convertir un mouvement de rotation de l'aimant permanent en un mouvement longitudinal de distraction, dans lequel l'élément d'entraînement (3) est conçu pour être actionné au moyen d'un champ magnétique d'une unité d'entraînement extracorporelle (7, 7', 7"), **caractérisé en ce qu'**une tige de flexion (35) est prévue pour une transmission de force de l'élément d'entraînement (3) à l'entraînement à broche (5).

2. Composant d'implant avec un dispositif de réglage selon la revendication 1, **caractérisé en ce qu'**une broche (50) de l'entraînement à broche (5) a un alésage central (51) ouvert d'un côté dans lequel est insérée la tige de flexion (35).

3. Composant d'implant avec un dispositif de réglage selon la revendication 2, **caractérisé en ce que** la tige de flexion (35) est couplée à son extrémité libre éloignée de l'élément d'entraînement (3) à une pièce de couplage (32) au niveau de la base de l'alésage central et la tige de flexion (35) a de préférence une tige rétrécie.

4. Composant d'implant avec un dispositif de réglage selon l'une quelconque des revendications 1 à 3, **caractérisé en ce que** la tige de flexion (35) a une longueur qui est de préférence d'au moins un tiers, de manière davantage préférée d'au moins la moitié, de la longueur de la broche d'entraînement (50), et est conçue en particulier pour s'adapter à un décalage angulaire allant jusqu'à +/- 5 degrés.

5. Composant d'implant avec un dispositif de réglage selon l'une quelconque des revendications 1 à 4, **caractérisé en ce que** la tige de flexion (35) est constituée d'un alliage de titane TiA16V4.

6. Système d'implant à réglage en longueur extracorporel, comprenant
- un composant d'implant (1) selon l'une quelconque des revendications précédentes ; et
- une unité d'entraînement extracorporelle (7, 7', 7") pour actionner l'élément d'entraînement (3) au moyen d'un champ magnétique, dans lequel l'unité d'entraînement extracorporelle (7, 7', 7") a un anneau magnétique extracorporel (8) monté rotatif avec un espace intérieur (80) qui est conçu pour recevoir l'os (90) à prolonger avec le dispositif de réglage (2), et un dispositif d'actionnement (75) qui est conçu pour faire tourner l'anneau magnétique extracorporel (8) dans son plan annulaire, dans lequel l'anneau magnétique extracorporel (8) est incorporé dans une conception d'aimant permanent et a un champ magnétique statique dirigé dans son espace intérieur qui est fixé dans l'anneau, et l'anneau magnétique extracorporel (8) est tourné mécaniquement par le dispositif d'actionnement (75).

7. Système d'implant selon la revendication 6, **caractérisé en ce que**
- le champ magnétique est concentré sur l'espace intérieur (80), et/ou
- le champ magnétique statique dirigé dans l'espace intérieur central est unidirectionnel et/ou homogène.

8. Système d'implant selon la revendication 6 ou la revendication 7, **caractérisé en ce que** l'anneau magnétique est divisible en au moins deux segments (81, 82) de manière à créer un accès ouvrable et fermable pour une partie de corps dotée du composant d'implant (1), en particulier une jambe ou un bras, à l'espace intérieur de l'anneau magnétique (8), dans lequel les segments (81, 82) peuvent être dépliés dans une position ouverte de l'anneau magnétique (8), dans lequel de préférence :
- les segments (81, 82) peuvent être dépliés, de préférence sans force magnétique, dans une position ouverte de l'anneau magnétique (8) ; et/ou
- un plan de séparation (83) entre les segments est sélectionné pour se prolonger parallèlement au champ magnétique statique dirigé ; et/ou
- les segments sont connectés à des moyens de connexion, dont au moins un (73) peut être ouvert et refermé et au moins un (74) peut être plié à la manière d'une charnière ; et/ou
- un dispositif de verrouillage (84) est prévu, lequel fixe l'anneau magnétique (8) avec ses segments (81, 82) dans une position d'ouverture de manière sécurisée en rotation ; et/ou
- un dispositif de sécurité est prévu sur l'anneau magnétique (8), qui est conçu, en particulier au moyen d'un boulon de verrouillage (84'), pour empêcher l'anneau magnétique (8) de se déplier en dehors de la position d'ouverture.

9. Système d'implant selon l'une quelconque des revendications 6 à 8, **caractérisé en ce que** l'anneau magnétique (8) est formé au moyen d'une pluralité de sous-aimants (85) de préférence identiques, dans lequel de préférence les sous-aimants sont des corps dipolaires magnétiques identiques (86), en particulier formés d'aimants en terres rares.

10. Système d'implant selon la revendication 9, **caractérisé en ce que** les sous-aimants (85) sont insérés le long de l'anneau magnétique (8) de manière régulière avec une orientation différente (87) de la magnétisation de manière angulairement fixe.

11. Système d'implant selon la revendication 10, **caractérisé en ce que** les sous-aimants (85) sont agencés de sorte que leurs champs magnétiques parasites se compensent mutuellement de manière maximale le long d'un plan de séparation (83) entre des segments (81, 82) de l'anneau magnétique (8), dans lequel les sous-aimants (85) sont de préférence agencés de sorte que leur champ magnétique parasite est nul le long d'un plan de séparation (83) entre des segments (81, 82) de l'anneau magnétique (8).

12. Système d'implant selon l'une quelconque des revendications 6 à 11, **caractérisé en ce qu'**un boîtier (71) est prévu pour l'unité d'entraînement (7, 7', 7"), lequel est de préférence doté d'un ou de plusieurs blindages (67, 68, 69) et entoure en particulier l'anneau magnétique (8) sans champs externes.

13. Système d'implant selon l'une quelconque des revendications 6 à 12, **caractérisé en ce que** l'unité d'entraînement (7, 7', 7") est dotée d'un dispositif de positionnement qui agit sur l'anneau magnétique (8), en particulier un compteur (66) indiquant les tours de l'anneau magnétique.

14. Système d'implant selon l'une quelconque des revendications 6 à 13, **caractérisé en ce que** l'unité d'entraînement est dotée d'un pied d'appui (6) servant de protection anti-basculement, lequel est de préférence doté d'un réceptacle (61, 62) à orientation directionnelle pour une partie de corps (9) dotée de la prothèse, en particulier un bras ou une jambe.
